# EUROPÄISCHE PATENTSCHRIFT

(11) **EP 3 801 930 B1**
(45) Veröffentlichungstag und Bekanntmachung des Hinweises auf die Patenterteilung: **10.05.2023**
(21) Anmeldenummer: 19737689.0
(22) Anmeldetag: 02.07.2019
(51) Int. Cl.: B06B 1/02, B06B 3/00, A61B 18/00

(54) **VERFAHREN ZUM BETRIEB EINES ELEKTROCHIRURGISCHEN SYSTEMS UND ULTRASCHALLGENERATOR**
METHOD FOR OPERATING AN ELECTROSURGICAL SYSTEM AND ULTRASOUND GENERATOR
PROCÉDÉ DE FONCTIONNEMENT D'UN SYSTÈME ÉLECTROCHIRURGICAL ET GÉNÉRATEUR D'ULTRASONS

(30) Priorität: 11.07.2018 DE 102018116771
(43) Veröffentlichungstag der Anmeldung: 14.04.2021
(73) Patentinhaber: OLYMPUS WINTER & IBE GMBH, 22045 Hamburg (DE)
(72) Erfinder: FAEHSING, Thomas, 12305 Berlin (DE)
(74) Vertreter: Noack, Andreas
(86) Internationale Anmeldenummer: PCT/EP2019/067680
(87) Internationale Veröffentlichungsnummer: WO 2020/011594

(56) Entgegenhaltungen:
- EP-A2- 1 199 047
- EP-A2- 1 199 048
- EP-A2- 1 835 622
- EP-B1- 1 199 048
- WO-A1-2016/091401
- WO-A2-2013/154925
- US-A1- 2004 102 709
- US-A1- 2010 312 111
- US-A1- 2013 310 689
- US-B2- 6 761 690
- US-B2- 8 845 537

## Beschreibung

Die Erfindung betrifft ein Verfahren zum Betrieb eines elektrochirurgischen Systems umfassend einen Ultraschallgenerator und ein Ultraschallinstrument, mit den Schritten: Ermitteln einer anfänglichen Resonanzfrequenz des Ultraschallinstruments durch den Ultraschallgenerator, Ansteuern des Ultraschallinstruments durch den Ultraschallgenerator mit einer Betriebsamplitude und einer Betriebsfrequenz, die der anfänglichen Resonanzfrequenz entspricht, Nachführen der Betriebsfrequenz des Ultraschallgenerators mit Änderungen der Resonanzfrequenz des Ultraschallinstruments, und Beenden der Ansteuerung des Ultraschallinstruments durch den Ultraschallgenerator.

Weiterhin betrifft die Erfindung einen Ultraschallgenerator.

In der modernen Elektrochirurgie werden neben reinen elektrochirurgischen Verfahren, in denen ein chirurgischer Effekt ausschließlich durch elektrische Ströme erreicht wird, auch Verfahren und Instrumente verwendet, bei denen ein hochfrequentes elektrisches Signal mittels eines Ultraschall-Transducers in eine Ultraschallschwingung umgesetzt wird, welche dann einen chirurgischen Effekt bewirkt. Dabei können ein durch Stromfluss bewirkter chirurgischer Effekt und ein durch Ultraschall bewirkter chirurgischer Effekt kombiniert werden.

Hierzu weisen entsprechende elektrochirurgische Systeme einen Ultraschallgenerator auf. Dieser Ultraschallgenerator erzeugt ein hochfrequentes elektrisches Signal, mit welchem das Ultraschallinstrument angesteuert wird. Das Ultraschallinstrument umfasst einen Ultraschall-Transducer, bei dem es sich in der Regel um ein piezoelektrisches Element handelt, welches bei Anliegen des hochfrequenten elektrischen Signals eine Ultraschallschwingung erzeugt. Die Ultraschallschwingung wird im Ultraschallinstrument auf ein Arbeitselement übertragen, welches bei Kontakt mit biologischem Gewebe einen chirurgischen Effekt bewirkt. Das Arbeitselement wird auch als Sonotrode bezeichnet.

Um einen wirksamen chirurgischen Effekt zu erzielen, wird der Ultraschall-Transducer in seiner Resonanzfrequenz betrieben. Diese Resonanzfrequenz hängt zum einen vom Typ des Ultraschallinstruments ab, zum anderen aber auch von Fertigungstoleranzen der Komponenten des Ultraschallinstruments und von externen Faktoren, wie z.B. der mechanischen Belastung während der Verwendung des Ultraschallinstruments. Die Resonanzfrequenz liegt in der Regel zwischen 10kHz und 100 kHz, beispielsweise bei 50kHz.

Um den Ultraschall-Transducer in seiner Resonanzfrequenz ansteuern zu können ist der Ultraschallgenerator eingerichtet, diese Resonanzfrequenz zu bestimmen. Dazu führt der Ultraschallgenerator bei unbelastetem Ultraschallinstrument einen sogenannten Scan-Vorgang durch, bei dem der Ultraschall-Transducer mit unterschiedlichen Frequenzen angesteuert wird. Da die Stärke der mechanischen Ultraschallschwingung nicht direkt gemessen werden kann, misst der Ultraschallgenerator ständig den Strom und die Spannung des hochfrequenten elektrischen Signals, und ermittelt die Phasenlage zwischen den beiden Größen. Aus der Phasenlage lässt sich ermitteln, ob die Frequenz des hochfrequenten elektrischen Signals der Resonanzfrequenz des Ultraschall-Transducers entspricht. Bei der Resonanzfrequenz sind Strom und Spannung des hochfrequenten Signals in Phase. Nebenresonanzen, bei denen Strom und Spannung ebenfalls in Phase sind, lassen sich dabei durch geeignete Zusatzbedingungen ausschließen.

Während der Anwendung ändert sich, beispielsweise durch wechselnde mechanische Belastung des Ultraschallinstruments oder Temperaturänderungen, die Resonanzfrequenz. Um die Betriebsfrequenz des hochfrequenten elektrischen Signals bei Änderungen der Resonanzfrequenz nachzuführen, misst der Ultraschallgenerator weiter die Phasenlage von Strom und Spannung und regelt die Betriebsfrequenz so, dass Strom und Spannung in Phase bleiben.

Wenn eine elektrochirurgische Prozedur bzw. ein Teilschritt einer elektrochirurgischen Prozedur abgeschlossen ist, wird die Ansteuerung des Ultraschallinstruments durch den Ultraschallgenerator beendet. Dazu wird in der Regel das hochfrequente elektrische Signal deaktiviert.

Hierbei tritt jedoch das Problem auf, dass in dem System aus Ultraschallgenerator und Ultraschallinstrument elektrische Energie und mechanische Energie gespeichert sind. Ohne das frequenzbestimmende hochfrequente elektrische Signal kann diese Energie Eigenschwingungen des Systems in anderen Frequenzen anregen, welche nicht auf den Ultraschall-Transducer abgestimmt sind. Hierdurch kann es zu Strom- und/oder Spannungsspitzen kommen, welche das Ultraschallinstrument schädigen oder gar zerstören können.

Die EP 1 199 048 A2 beschreibt ein Verfahren zum Betrieb eines Ultraschallsystems, mit welchem eine für den Betrieb des Systems gewünschte Resonanzfrequenz einer Ultraschallklinge beim Start des Systems gemessen und dann mittels einer phasengekoppelten Regelschleife verfolgt wird.

Die EP 1 835 622 A2 beschreibt einen Ultraschallgenerator sowie ein Verfahren zum Ultraschallschweißen, bei welchem eine Anregungsamplitude des Generators in Form einer Rampe reduziert wird, um Komponenten des Ultraschallsystems zu schonen.

Die WO 2016/091401 A1 beschreibt einen elektrochirurgischen Generator, ein Steuergerät und ein Steuerverfahren, bei welchem auch in Behandlungspausen eine minimale Anregungsamplitude ausgegeben wird, um eine Resonanzfrequenz des elektrochirurgischen Systems zu halten.

Die Anmeldung US 2013/310689 A1 beschreibt ein Ultraschalldiagnosegerät.

Es besteht daher die Aufgabe der Erfindung darin, ein Verfahren zum Betrieb eines elektrochirurgischen Systems sowie einen Ultraschallgenerator bereitzustellen, welches bezüglich der beschriebenen Problematik verbessert sind.

Die Aufgabe wird gemäß eines Aspekts der Erfindung gelöst durch ein Verfahren zum Betrieb eines elektrochirurgischen Systems umfassend einen Ultraschallgenerator und ein Ultraschallinstrument, mit den Schritten: Ermitteln einer anfänglichen Resonanzfrequenz des Ultraschallinstruments durch den Ultraschallgenerator, Ansteuern des Ultraschallinstruments durch den Ultraschallgenerator mit einer Betriebsamplitude und einer Betriebsfrequenz, die der anfänglichen Resonanzfrequenz entspricht, Nachführen der Betriebsfrequenz des Ultraschallgenerators mit Änderungen der Resonanzfrequenz des Ultraschallinstruments, und Beenden der Ansteuerung des Ultraschallinstruments durch den Ultraschallgenerator, welches dadurch weitergebildet ist, dass zum Beenden der Ansteuerung des Ultraschallinstruments durch den Ultraschallgenerator in einer Abklingphase die Betriebsamplitude des Ultraschallgenerators mit einer vorgegebenen oder vorgebbaren Änderungsrate auf null reduziert wird.

Durch das erfindungsgemäße Verfahren wird erreicht, dass die Schwingfrequenz des Systems aus Ultraschallgenerator und Ultraschallinstruments während der Beendigung der Ansteuerung durch die Betriebsfrequenz des Ultraschallgenerators vorgegeben ist, und somit eine Anregung von anderen Eigenschwingungen nicht auftreten kann. Die in dem System gespeicherte Energie wird dabei durch ohmsche Verluste und mechanische Reibung abgebaut, ohne dass es zu Strom- oder Spannungsspitzen kommt.

Oftmals geht das Beenden der Ansteuerung des Ultraschallinstruments damit einher, dass das Ultraschallinstrument von dem zu behandelnden Gewebe entfernt wird. Dadurch kann sich die mechanische Belastung des Ultraschallinstruments und somit auch die Resonanzfrequenz des Ultraschall-Transducers kurzfristig ändern

Daher wird die Betriebsfrequenz des Ultraschallgenerators zumindest in einem ersten Abschnitt der Abklingphase mit Änderungen der Resonanzfrequenz des Ultraschallinstruments nachgeführt. Somit ist auch bei einer Änderung der Resonanzfrequenz des Ultraschall-Transducers während der Abklingphase sichergestellt, dass die Betriebsfrequenz der Resonanzfrequenz entspricht.

Um die Betriebsfrequenz des Ultraschallgenerators mit der Resonanzfrequenz des Ultraschall-Transducers nachführen zu können, ist allerdings eine verlässliche Bestimmung der Phasenlage zwischen dem Strom und der Spannung des hochfrequenten elektrischen Signals erforderlich, wozu eine ausreichende Betriebsamplitude des Ultraschallgenerators benötigt wird. Unterschreitet die Betriebsamplitude diesen Wert, so sind die Phasenbestimmung und die Frequenznachführung nicht mehr möglich.

Daher wird die Betriebsfrequenz des Ultraschallgenerators zumindest in einem zweiten Abschnitt der Abklingphase auf einem konstanten Wert gehalten. Somit kann auch bei geringer Betriebsamplitude des Ultraschallgenerators ein unkontrolliertes Schwingungsverhalten des Systems verhindert werden.

Der Übergang zwischen dem ersten Abschnitt und dem zweiten Abschnitt der Abklingphase kann dabei besonders vorzugsweise erfolgen, wenn die Betriebsamplitude des Ultraschallgenerators einen vorgegebenen oder vorgebbaren Grenzwert unterschreitet.

In einer alternativen Ausführungsform der Erfindung kann der Übergang zwischen dem ersten Abschnitt und dem zweiten Abschnitt der Abklingphase zu einer vorgegebenen oder vorgebbaren Zeit nach dem Einleiten der Abklingphase erfolgen.

Die Betriebsfrequenz des Ultraschallgenerators kann in dem zweiten Abschnitt der Abklingphase auf einem Wert gehalten werden, welcher einem Mittelwert der Betriebsfrequenz des Ultraschallgenerators in einem vorgegebenen oder vorgebbaren Zeitabschnitt vor Beginn des zweiten Abschnitts der Abklingphase entspricht. Hierbei ist die Wahrscheinlichkeit besonders groß, dass die Betriebsfrequenz der tatsächlichen Resonanzfrequenz besonders gut entspricht. Als Mittelwert kann dabei im Sinne der Erfindung entweder ein arithmetischer Mittelwert Anwendung finden, oder ein gleitender Mittelwert, ein Medianwert, oder ein Modalwert.

Der vorgegebene oder vorgebbare Zeitabschnitt kann dabei vorzugsweise vollständig vor dem Beginn der Abklingphase liegen.

Die Aufgabe wird gemäß eines weiteren Aspekts der Erfindung gelöst durch einen Ultraschallgenerator eines elektrochirurgischen Systems umfassend einen

Ultraschallgenerator und ein Ultraschallinstrument, wobei der Ultraschallgenerator eingerichtet ist, das elektrochirurgische System gemäß eines Verfahrens nach den obigen Ausführungen zu betreiben. Bezüglich der hierdurch erreichten Wirkungen und Vorteile wird ausdrücklich auf das oben gesagte verwiesen.

Die Erfindung wird nachfolgend anhand einiger beispielhafter Darstellungen näher erläutert. Die dargestellten Ausführungsbeispiele dienen hierbei lediglich zum besseren Verständnis der Erfindung, ohne diese einzuschränken.

Es zeigen:
Fig. 1: ein elektrochirurgisches System,
Fig. 2: den Aufbau eines Ultraschallinstruments,
Fig. 3: den Phasengang eines Ultraschall-Transducers,
Fig. 4: den schematischen Aufbau eines Utraschallgenerators,
Fig. 5: Betriebsfrequenz und Betriebsamplitude des Ultraschallgenerators in der Abklingphase.

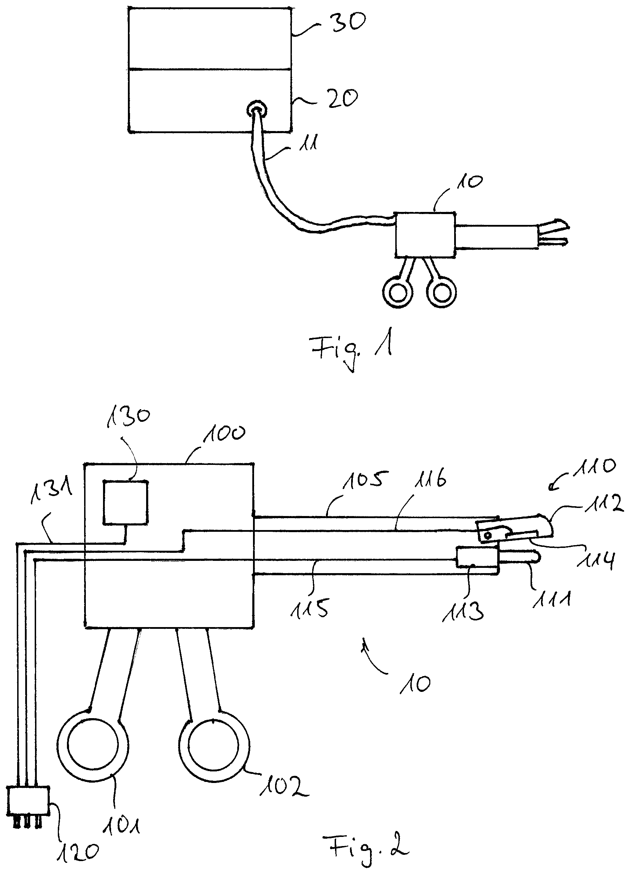

Figur 1 zeigt ein elektrochirurgisches System mit einem Ultraschallinstrument 10, einem Ultraschallgenerator 20, sowie einem Hochfrequenzgenerator 30. Das Ultraschallinstrument 10 ist über ein Kabel 11 mit dem Ultraschallgenerator 20 verbunden.

Das Ultraschallinstrument 10 kann beispielsweise eine kombinierte Hochfrequenz- und Ultraschallzange sein, wie sie beispielsweise unter dem Namen THUNDERBEAT von der Olympus Corporation vertrieben wird.

Im Betrieb erzeugt der Ultraschallgenerator 20 ein erstes hochfrequentes elektrisches Signal, welches über das Kabel 11 an das Ultraschallinstrument 10 übertragen wird, und dort von einem nicht dargestellten Ultraschall-Transducer in eine Ultraschall-Schwingung umgesetzt wird. Die Ultraschallschwingung wird in eine nicht dargestellte Sonotrode eingekoppelt, welche direkt oder indirekt mit zu behandelndem Gewebe in Kontakt gebracht werden kann.

Der Hochfrequenzgenerator 30 erzeugt im Betrieb ein zweites hochfrequentes elektrisches Signal, welches über eine interne Verbindung an den Ultraschallgenerator 20 und von dort ebenfalls über das Kabel 11 an das Ultraschallinstrument 10 übertragen wird. Im Ultraschallinstrument 10 wird das zweite hochfrequente elektrische Signal an eine oder mehrere Elektroden geführt, welche direkt oder indirekt mit zu behandelndem Gewebe in Kontakt gebracht werden können.

In Figur 2 ist der Aufbau des Ultraschallinstruments 10 näher dargestellt, wobei die Darstellung nicht maßstabsgetreu und stark vereinfacht ist.

Das Ultraschallinstrument 10 besteht aus einem Hauptkörper 100 mit Griffhebeln 101,102. An den Hauptkörper 100 schließt ein Schaft 105 an, an dessen distalem Ende ein Zangenmaul 110 angeordnet ist.

Das Zangenmaul 110 umfasst hier eine feststehende Branche, welche durch eine Sonotrode 111 gebildet ist, und eine bewegliche Branche 112. Die Sonotrode 111 ist mit einem Ultraschall-Transducer 113 gekoppelt. An der beweglichen Branche 112 ist eine Elektrode 114 angeordnet.

Die bewegliche Branche 112 kann durch Betätigen eines der Griffhebel 101,102 in Richtung der Sonotrode 111 bewegt werden, so dass sich das Zangenmaul 110 schließt. Ein nicht dargestellter Abschnitt von menschlichem oder tierischem Gewebe, der in dem geschlossenen Zangenmaul 110 eingeklemmt ist, kann dann durch Aktivieren der Sonotrode 111 und/oder der Elektrode 114 behandelt werden.

Zur Aktivierung der Sonotrode 111 wird ein erstes hochfrequentes elektrisches Signal von dem Ultraschallgenerator 20 dem Ultraschall-Transducer 113 zugeführt. Dieser setzt das Signal in eine Ultraschallschwingung um und überträgt diese an die Sonotrode 111. Die mechanische Bewegung der Sonotrode 111, die in engem Kontakt mit dem zu behandelnden Gewebe steht, bewirkt dann einen chirurgischen Effekt in dem Gewebe, welcher je nach Ausführung der Sonotrode und gewünschtem Ergebnis variieren kann.

Zur Aktivierung der Elektrode 114 wird dieser ein zweites hochfrequentes elektrisches Signal von dem elektrochirurgischen Generator 30 zugeführt.

Die Zuführung der elektrischen Signale erfolgt über Leitungen 115,116. Die Leitungen 115,116 enden in einem Stecker 120, der mit dem Ultraschallgenerator 20 verbunden werden kann.

Das Ultraschallinstrument 10 umfasst weiterhin ein Speicherelement 130, dessen Funktion später erläutert wird. Das Speicherelement 130 ist über eine Leitung 131 ebenfalls mit dem Stecker 120 verbunden.

Um einen optimalen chirurgischen Effekt zu erzielen ist es wünschenswert, dass der Ultraschallgenerator 20 den Ultraschall-Transducer 113 in seiner mechanischen Resonanzfrequenz ansteuert. Diese mechanische Resonanzfrequenz hängt jedoch von diversen Parametern ab, beispielsweise Fertigungstoleranzen des Ultraschall-Transducers 113, aber auch Art und Menge des im Zangenmaul 110 gegriffenen Gewebes und dem Anpressdruck der beweglichen Branche 112, sowie etlichen weiteren Parametern wie der Temperatur des Ultraschall-Transducers und dem Verschleiß- und/oder Verschmutzungsgrad des Ultraschallinstruments. Diese und weitere beeinflussende Parameter sind dem Fachmann bekannt.

Um die aktuelle Resonanzfrequenz des Ultraschall-Transducers 113 zu Beginn einer Aktivierungsphase zu bestimmen, führt der Ultraschallgenerator 20 einen sogenannten Scan durch, wobei der Ultraschall-Transducer nacheinander mit mehreren Frequenzen angesteuert wird und der Verlauf der Phasenlage von Strom und Spannung des ersten hochfrequenten elektrischen Signals gemessen wird.

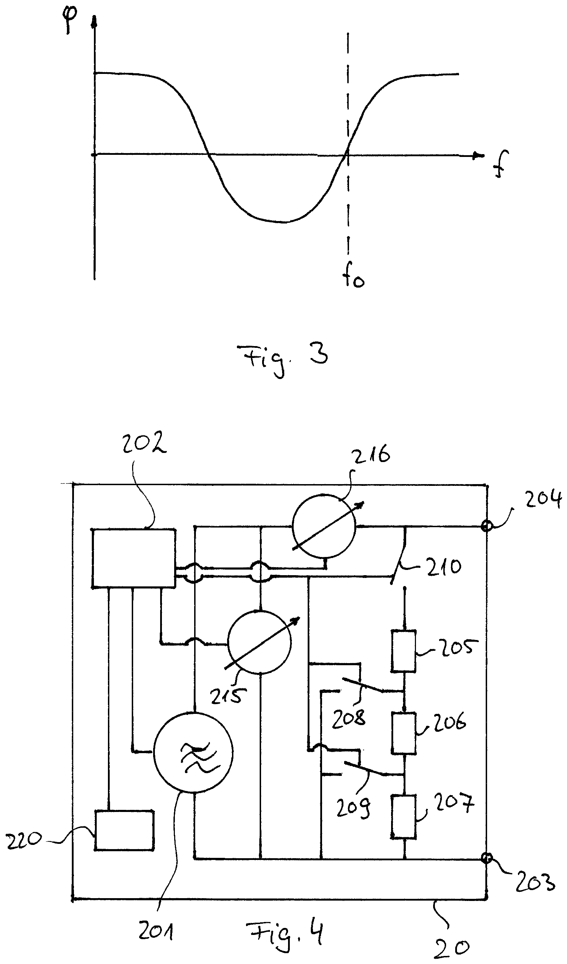

Der Verlauf der Phasenlage ϕ von Strom und Spannung in Abhängigkeit von der Betriebsfrequenz f ist in Figur 3 dargestellt. Es ist zu erkennen, dass bei niedriger Betriebsfrequenz zunächst eine positive Phasenlage besteht, also der Strom der Spannung voreilt. Bei steigender Betriebsfrequenz sinkt die Phase ab, durchschreitet den Nullpunkt, und wird zunächst negativ. In diesem Frequenzbereich eilt die Spannung dem Strom vor. Bei Annäherung an die Resonanzfrequenz f₀ steigt die Phase ϕ wieder an und durchschreitet bei Erreichen der Resonanzfrequenz f₀ erneut den Nullpunkt, um bei weiter steigenden Betriebsfrequenzen positiv zu werden. Hier eilt also wieder der Strom der Spannung vor.

Der Phasenverlauf bei niedrigen Betriebsfrequenzen ist durch eine baulich bedingte Parallelkapazität bestimmt. Hierbei handelt es sich hauptsächlich um die kapazitive Wirkung von Kontaktierungsflächen, die auf den Piezokristallen des Ultraschall-Transducers aufgedampft sind.

In Figur 4 ist der Aufbau des Ultraschallgenerators 20 schematisch dargestellt, soweit er für das Verständnis der Erfindung relevant ist.

Ein Oszillator 201 erzeugt ein hochfrequentes elektrisches Signal mit steuerbarer Frequenz. Die Betriebsfrequenz des Oszillators 201 wird von einer Steuerung 202 gesteuert. Das hochfrequente elektrische Signal wird an Ausgangsklemmen 203,204 bereitgestellt, welche mit Kontakten des Steckers 120 des Ultraschallinstruments 10 verbunden werden können.

Zwischen den Ausgangsklemmen 203,204 sind Induktivitäten 205,206,207 angeordnet, welche durch Schalter 208,209,210 zu- oder abgeschaltet werden können. Die Induktivitäten 205,206,207 dienen dazu, die Phasenverschiebung zwischen Strom und Spannung des hochfrequenten elektrischen Signals, welche durch die Parallelkapazität des Ultraschall-Transducers 113 verursacht ist, zu kompensieren. Die Schalter 208,209,210 werden durch die Steuerung 202 kontrolliert.

Je nach Bauart eines angeschlossenen Ultraschallinstruments werden alle oder einzelne der Induktivitäten 205,206,207 durch die Steuerung aktiviert. Die Erkennung eines angeschlossenen Ultraschallinstruments erfolgt mittels bekannter Methoden zur Instrumentenerkennung, die hier nicht näher erläutert werden müssen. Dabei können beispielsweise im Speicher 130 des Ultraschallinstruments 10 abgelegte Informationen ausgewertet werden.

Über Sensoren 215,216 werden der Strom und die Spannung des hochfrequenten elektrischen Signals in kurzen Abständen abgetastet. Aus den Abtastwerten ermittelt die Steuerung 202 die Phasenlage zwischen Strom und Spannung und regelt die Betriebsfrequenz des Oszillators 201 so, dass Strom und Spannung in Phase sind, um so den Ultraschall-Transducer 113 in seiner Resonanzfrequenz anzusteuern.

Wird nun zum Beenden der Ansteuerung des Ultraschallinstruments 10 der Oszillator 201 abgeschaltet, so bilden der Ultraschall-Transducer 113, das Kabel 10, und die zugeschalteten Induktivitäten 205,206,207 einen elektrischen Schwingkreis, welcher durch die in dem System gespeicherte Energie angeregt wird. Da die Eigenfrequenz dieses Schwingkreises nicht auf des Ultraschall-Transducer 113 abgestimmt ist, kann es zu Strom- und/oder Spannungsspitzen kommen, welche den Ultraschall-Transducer 113 schädigen oder zerstören können.

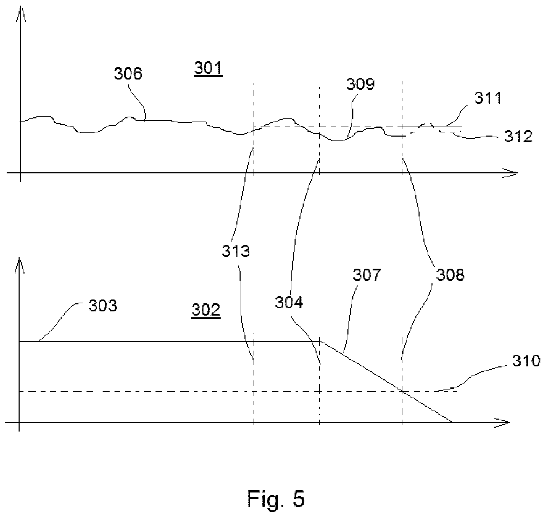

Um dies zu verhindern wird die Betriebsamplitude des Ultraschallgenerators 20 bei der Beendigung der Aktivierung des Ultraschallinstruments 10 kontrolliert reduziert, dies ist in Figur 5 dargestellt. Dabei zeigt das obere Diagramm 301 den Verlauf der Betriebsfrequenz des Ultraschallgenerators über die Zeit, während das untere Diagramm 302 den Verlauf der Betriebsamplitude des Ultraschallgenerators über die Zeit zeigt.

Die Betriebsamplitude des Ultraschallgenerators 20 ist zunächst konstant (Linie 303), bis bei einem Zeitpunkt 304 die Abklingphase eingeleitet wird, beispielsweise indem ein Benutzer des elektrochirurgischen Systems einen Aktivierungsschalter loslässt, oder wenn ein vorgegebenes Abbruchkriterium für die Behandlung erreicht ist. Dies kann beispielsweise eine vorgegebene Behandlungszeit oder das Erreichen bestimmter elektrischer und/oder mechanischer Impedanzwerte oder einer vorgegebenen Instrumenten- oder Gewebetemperatur sein. Bis zu dem Zeitpunkt 304 wird die Betriebsfrequenz des Ultraschallgenerators 20 mit der Resonanzfrequenz des Ultraschall-Transducers 113 nachgeführt (Linie 306).

Ab dem Zeitpunkt 304 wird die Ausgangsamplitude des Ultraschalgenerators 20 mit einer vorgegebenen Änderungsrate geändert, vorzugsweise reduziert (Linie 307). Während in Figur 5 eine lineare Reduktion der Betriebsamplitude dargestellt ist, kann diese gleichermaßen auch exponentiell, S-förmig, oder nach einer anderen Zeitfunktion erfolgen.

Bis zu einem Zeitpunkt 308 wird die Betriebsfrequenz des Ultraschallgenerators 20 weiter mit der Resonanzfrequenz des Ultraschall-Transducers 113 nachgeführt (Linie 309). Zum Zeitpunkt 308 unterschreitet die Betriebsamplitude des Ultraschallgenerators 20 eine minimale Betriebsamplitude (Linie 310), die zur Nachführung der Betriebsfrequenz mit der Resonanzfrequenz erforderlich ist. Daher wird ab dem Zeitpunkt 308 die Betriebsfrequenz des Ultraschallgenerators auf einem festen Wert gehalten (Linie 311), unabhängig von tatsächlichen Schwankungen der Resonanzfrequenz (gestrichelte Linie 312).

Der Wert der Betriebsfrequenz des Ultraschallgenerators 20 nach dem Zeitpunkt 308 wird anhand eines Mittelwerts festgelegt, welcher beispielsweise zwischen dem Zeitpunkt 313, welcher vor dem Zeitpunkt 304 liegt, und dem Zeitpunkt 304 bestimmt wird. Der Zeitpunkt 313 kann auch nach dem Zeitpunkt 304, aber vor dem Zeitpunkt 308 liegen, oder dem Zeitpunkt 308 entsprechen.

Die Lage des Zeitpunkts 308, zu dem die Nachführung der Betriebsfrequenz beendet wird, kann anstelle von einer Unterschreitung der minimalen Betriebsamplitude auch durch einen festen Zeitabstand zu dem Zeitpunkt 304 festgelegt sein.

Obwohl die Erfindung vorangehend anhand eines zangenartigen kombinierten Ultraschall- und Hochfrequenzinstruments 10 beschrieben ist, kann sie selbstverständlich auch bei einem reinen Ultraschallinstrument angewendet werden. Der Hochfrequenzgenerator 30 kann dabei entfallen. Ebenso kann die Erfindung mit anderen Instrumentenformen wie z.B. Klingen, Spateln oder Haken verwendet werden.

Ebenso ist es denkbar, dass Ultraschallgenerator 20 und Hochfrequenzgenerator 30 als ein einziges Gerät ausgeführt sind.

In der Speicher 130 des Ultraschallinstruments 10 können neben Daten zur Instrumentenerkennung auch weitere Daten abgelegt sein, welche der Ultraschallgenerator 20 bei der Bestimmung der Resonanzfrequenz verwendet. Beispielsweise kann eine werksseitig ermittelte Resonanzfrequenz bereits auf dem Speicher 130 abgelegt sein und dann vom Ultraschallgenerator 20 ausgelesen werden. Alternativ kann in dem Speicher 130 auch ein Frequenzbereich hinterlegt sein, in welchem die Resonanzfrequenz enthalten ist, so dass der Ultraschallgenerator 20 den Scan-Vorgang auf diesen Frequenzbereich beschränken kann.

## Patentansprüche

1. Verfahren zum Betrieb eines elektrochirurgischen Systems umfassend einen Ultraschallgenerator (20) und ein Ultraschallinstrument (10), mit den Schritten:
- Ermitteln einer anfänglichen Resonanzfrequenz des Ultraschallinstruments (10) durch den Ultraschallgenerator (20),
- Ansteuern des Ultraschallinstruments (10) durch den Ultraschallgenerator (20) mit einer Betriebsamplitude und einer Betriebsfrequenz, die der anfänglichen Resonanzfrequenz entspricht,
- Nachführen der Betriebsfrequenz des Ultraschallgenerators (20) mit Änderungen der Resonanzfrequenz des Ultraschallinstruments (10), und
- Beenden der Ansteuerung des Ultraschallinstruments (10) durch den Ultraschallgenerator (20),
wobei zum Beenden der Ansteuerung des Ultraschallinstruments (10) durch den Ultraschallgenerator (20) in einer Abklingphase die Betriebsamplitude des Ultraschallgenerators (20) mit einer vorgegebenen oder vorgebbaren Änderungsrate auf null reduziert wird, **dadurch gekennzeichnet, dass** die Betriebsfrequenz des Ultraschallgenerators (20) zumindest in einem ersten Abschnitt der Abklingphase mit Änderungen der Resonanzfrequenz des Ultraschallinstruments (10) nachgeführt wird, und dass die Betriebsfrequenz des Ultraschallgenerators (20) zumindest in einem zweiten Abschnitt der Abklingphase auf einem konstanten Wert gehalten wird.

2. Verfahren nach Anspruch 1, **dadurch gekennzeichnet, dass** der Übergang zwischen dem ersten Abschnitt und dem zweiten Abschnitt der Abklingphase erfolgt, wenn die Betriebsamplitude des Ultraschallgenerators (20) einen vorgegebenen oder vorgebbaren Grenzwert unterschreitet.

3. Verfahren nach Anspruch 1, **dadurch gekennzeichnet, dass** der Übergang zwischen dem ersten Abschnitt und dem zweiten Abschnitt der Abklingphase zu einer vorgegebenen oder vorgebbaren Zeit nach dem Einleiten der Abklingphase erfolgt.

4. Verfahren nach einem der vorangegangenen Ansprüche, **dadurch gekennzeichnet, dass** die Betriebsfrequenz des Ultraschallgenerators in dem zweiten Abschnitt der Abklingphase auf einem Wert gehalten wird, welcher einem Mittelwert der Betriebsfrequenz des Ultraschallgenerators (20) in einem vorgegebenen oder vorgebbaren Zeitabschnitt vor Beginn des zweiten Abschnitts der Abklingphase entspricht.

5. Verfahren nach Anspruch 4, **dadurch gekennzeichnet, dass** der vorgegebene oder vorgebbare Zeitabschnitt vollständig vor dem Beginn der Abklingphase liegt.

6. Ultraschallgenerator (20) eines elektrochirurgischen Systems umfassend einen Ultraschallgenerator (20) und ein Ultraschallinstrument (10), **dadurch gekennzeichnet, dass** der Ultraschallgenerator (20) eingerichtet ist, das elektrochirurgische System gemäß eines Verfahrens nach einem der Ansprüche 1 bis 5 zu betreiben.

## Claims

1. Method of operating an electrosurgical system comprising an ultrasound generator (20) and an ultrasound instrument (10), comprising the steps of:
- Determining an initial resonance frequency of the ultrasound instrument (10) by the ultrasound generator (20),
- Driving the ultrasound instrument (10) by the ultrasound generator (20) with an operating amplitude and an operating frequency corresponding to the initial resonance frequency,
- Adjusting the operating frequency of the ultrasound generator (20) with changes in the resonance frequency of the ultrasound instrument (10), and
- Terminating driving of the ultrasound instrument (10) by the ultrasound generator (20),
wherein for terminating the control of the ultrasound instrument (10) by the ultrasound generator (20), in a decay phase, the operating amplitude of the ultrasound generator (20) is reduced to zero at a predetermined or predeterminable rate of change, **characterized in that** the operating frequency of the ultrasound generator (20) is adjusted with changes of the resonance frequency of the ultrasound instrument (10) at least in a first section of the decay phase, and that the operating frequency of the ultrasound generator (20) is kept at a constant value at least in a second section of the decay phase.

2. Method according to claim 1, **characterized in that** the transition between the first section and the second section of the decay phase takes place when the operating amplitude of the ultrasonic generator (20) falls below a predetermined or predeterminable limit value.

3. Method according to claim 1, **characterized in that** the transition between the first section and the second section of the decay phase takes place at a predetermined or predeterminable time after the initiation of the decay phase.

4. Method according to any one of the preceding claims, **characterized in that** the operating frequency of the ultrasound generator in the second section of the decay phase is maintained at a value which corresponds to an average value of the operating frequency of the ultrasound generator (20) in a predetermined or predeterminable time period before the start of the second section of the decay phase.

5. Method according to claim 4, **characterized in that** the predetermined or predeterminable time period is entirely before the beginning of the decay phase.

6. Ultrasound generator (20) of an electrosurgical system comprising an ultrasound generator (20) and an ultrasound instrument (10), **characterized in that** the ultrasound generator (20) is configured to operate the electrosurgical system according to a method according to any one of claims 1 to 5.

## Revendications

1. Procédé de fonctionnement d'un système électrochirurgical comprenant un générateur d'ultrasons (20) et un instrument à ultrasons (10), comprenant les étapes de :
- Détermination d'une fréquence de résonance initiale de l'instrument à ultrasons (10) par le générateur d'ultrasons (20),
- Commande de l'instrument à ultrasons (10) par le générateur d'ultrasons (20) avec une amplitude de fonctionnement et une fréquence de fonctionnement qui correspondent à la fréquence de résonance initiale,
- Adaptation de la fréquence de fonctionnement du générateur d'ultrasons (20) aux changements de la fréquence de résonance de l'instrument à ultrasons (10), et
- Terminaison de la commande de l'instrument à ultrasons (10) par le générateur d'ultrasons (20),
dans lequel, pour la terminaison de la commande de l'instrument à ultrasons (10) par le générateur d'ultrasons (20) dans une phase de décroissance, l'amplitude de fonctionnement du générateur d'ultrasons (20) est réduite à zéro avec un taux de variation prédéterminé ou prédéterminable, **caractérisé en ce que** la fréquence de fonctionnement du générateur d'ultrasons (20) est adaptée aux variations de la fréquence de résonance de l'instrument à ultrasons (10) au moins dans une première section de la phase de décroissance, et **en ce que** la fréquence de fonctionnement du générateur d'ultrasons (20) est maintenue à une valeur constante au moins dans une deuxième section de la phase de décroissance.

2. Procédé selon la revendication 1, **caractérisé en ce que** la transition entre la première section et la deuxième section de la phase de décroissance a lieu lorsque l'amplitude de fonctionnement du générateur d'ultrasons (20) est inférieure à une valeur limite prédéterminée ou prédéterminable.

3. Procédé selon la revendication 1, **caractérisé en ce que** la transition entre la première section et la deuxième section de la phase de décroissance a lieu à un moment prédéterminé ou prédéterminable après l'initiation de la phase de décroissance.

4. Procédé selon l'une quelconque des revendications précédentes, **caractérisé en ce que** la fréquence de fonctionnement du générateur d'ultrasons est maintenue, dans la deuxième portion de la phase de décroissance, à une valeur correspondant à une valeur moyenne de la fréquence de fonctionnement du générateur d'ultrasons (20) dans un intervalle de temps prédéterminé ou prédéterminable avant le début de la deuxième portion de la phase de décroissance.

5. Procédé selon la revendication 4, **caractérisé en ce que** l'intervalle de temps prédéterminé ou prédéterminable est entièrement antérieur au début de la phase de décroissance.

6. Générateur d'ultrasons (20) d'un système électrochirurgical comprenant un générateur d'ultrasons (20) et un instrument à ultrasons (10), **caractérisé en ce que** le générateur d'ultrasons (20) est adapté pour faire fonctionner le système électrochirurgical selon un procédé selon l'une quelconque des revendications 1 à 5.
